# EUROPÄISCHE PATENTSCHRIFT

(11) **EP 1 501 820 B1**
(45) Veröffentlichungstag und Bekanntmachung des Hinweises auf die Patenterteilung: **31.05.2006**
(21) Anmeldenummer: 03725028.9
(22) Anmeldetag: 15.04.2003
(51) Int. Cl.: C07D 333/38, A01N 43/10

(54) **METHYLTHIOPHENCARBOXANILIDE**
METHYLTHIOPHENE CARBOXANILIDES
METHYLTHIOPHENOCARBOXA NILIDES

(30) Priorität: 24.04.2002 DE 10218231
(43) Veröffentlichungstag der Anmeldung: 02.02.2005
(73) Patentinhaber: Bayer CropScience AG, 40789 Monheim (DE)
(72) Erfinder: DUNKEL, Ralf, 40789 Monheim (DE); RIECK, Heiko, 69110 Ste. Foy lés Lyon (FR); ELBE, Hans-Ludwig, 42329 Wuppertal (DE); WACHENDORFF-NEUMANN, Ulrike, 56566 Neuwied (DE); MAULER-MACHNIK, Astrid, 42799 Leichlingen (DE); KUCK, Karl-Heinz, 40764 Langenfeld (DE)
(86) Internationale Anmeldenummer: PCT/EP2003/003894
(87) Internationale Veröffentlichungsnummer: WO 2003/091240

(56) Entgegenhaltungen:
- EP-A- 0 741 131
- WO-A-02/08197
- WO-A-93/11097
- US-A- 5 438 070
- US-B1- 6 369 093
- WHITE, G. A. ET AL: "Thiophene carboxamide fungicides: structure-activity relationships with the succinate dehydrogenase complex from wild-type and carboxin-resistant mutant strains of Ustilago maydis" PESTICIDE BIOCHEMISTRY AND PHYSIOLOGY (1980), 14(1), 26-40 , XP009016749
- WHITE, G. A. ET AL: "Thiophene carboxamide fungicides: structure-activity relationships with the succinate dehydrogenase complex from wild-type and carboxin-resistant mutant strains of Aspergillus nidulans" PESTICIDE BIOCHEMISTRY AND PHYSIOLOGY (1986), 25(2), 188-204 , XP009016748

## Beschreibung

Die vorliegende Erfindung betrifft neue Methylthiophencarboxanilide, mehrere Verfahren zu deren Herstellung und deren Verwendung zur Bekämpfung von schädlichen Mikroorganismen im Pflanzenschutz und Materialschutz.

Es ist bereits bekannt geworden, dass zahlreiche Carboxanilide fungizide Eigenschaften besitzen (vgl. z.B. WO 93/11097, Can. Pestic. Biochem. Physiol. 1980, 14, 26-40, Can. Pestic. Biochem. Physiol. 1986, 25, 188-204, JP 2001-72507, JP 2001-72510 oder EP-A 0 545 099). Die Wirksamkeit der dort beschriebenen Stoffe ist gut, lässt aber bei niedrigen Aufwandmengen in manchen Fällen zu wünschen übrig.

Es wurden nun neue Methylthiophencarboxanilide der Formel (I) 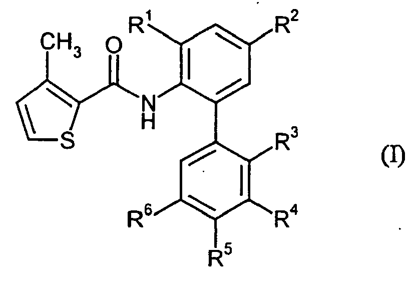 gefunden, in welcher
- R¹ und R²: gleich oder verschieden sind und unabhängig voneinander für Wasserstoff oder Fluor stehen,
- R³, R⁴ und R⁶: gleich oder verschieden sind und unabhängig voneinander für Wasserstoff, Halogen, C₁-C₆-Alkyl oder C₁-C₄-Halogenalkyl mit 1 bis 5 Halogenatomen stehen,
- R⁵: für Wasserstoff, Halogen, Cyano, Nitro, C₁-C₆-Alkyl, C₂-C₆-Alkenyl, C₃-C₆₋Cycloalkyl, C₁-C₄-Alkoxy, C₁-C₄-Alkylthio, C₁-C₄-Alkylsulfonyl, C₁-C₄₋Halogenalkyl mit 1 bis 5 Halogenatomen, C₁-C₄-Halogenalkoxy mit 1 bis 5 Halogenatomen, C₁-C₄-Halogenalkylthio mit 1 bis 5 Halogenatomen oder C₁₋C₄-Halogenalkylsulfonyl mit 1 bis 5 Halogenatomen steht,
wobei R³, R⁴, R⁵ und R⁶ nicht gleichzeitig für Wasserstoff stehen.
Weiterhin wurde gefunden, dass man Methylthiophencarboxanilide der Formel (I) erhält, indem man
a) Methylthiophencarbonsäurehalogenide der Formel (II) 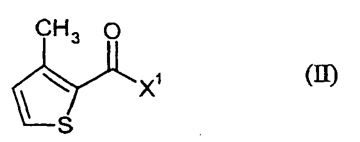 in welcher
   - X¹: für Halogen steht,
   mit einem Anilinderivat der Formel (III) 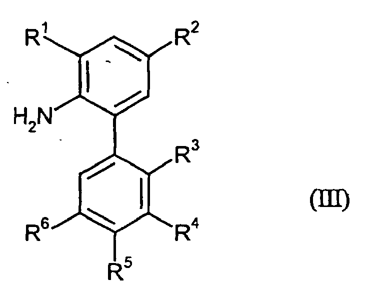 in welcher
   R¹, R², R³, R⁴, R⁵ und R⁶ die oben angegebenen Bedeutungen haben,
   gegebenenfalls in Gegenwart eines Säurebindemittels und gegebenenfalls in Gegenwart eines Verdünnungsmittels umsetzt, oder
b) Methylthiophencarboxhalogenanilide der Formel (IV) 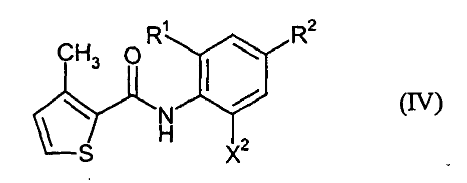 in welcher
   - R¹ und R²: die oben angegebenen Bedeutungen haben und
   - X²: für Brom oder Iod steht,
   mit einer Boronsäure der Formel (V) 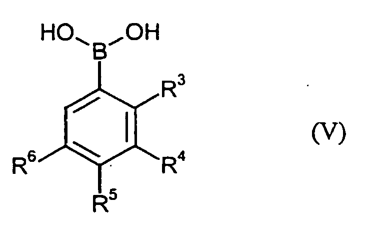 in welcher
   R³, R⁴, R⁵ und R⁶ die oben angegebenen Bedeutungen haben,
   in Gegenwart eines Katalysators, gegebenenfalls in Gegenwart eines Säurebindemittels und gegebenenfalls in Gegenwart eines Verdünnungsmittels
   umsetzt.
   Schließlich wurde gefunden, dass die neuen Methylthiophencarboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.
   Überraschenderweise zeigen die erfindungsgemäßen Methylthiophencarboxanilide der Formel (I) eine wesentlich bessere fungizide Wirksamkeit als die konstitutionell ähnlichsten, vorbekannten Wirkstoffe gleicher Wirkungsrichtung.
   Die erfindungsgemäßen Methylthiophencarboxanilide sind durch die Formel (I) allgemein definiert.
   Bevorzugt sind Methylthiophencarboxanilide der Formel (I) sind, in welcher
   - R¹ und R²: gleich oder verschieden sind und unabhängig voneinander für Wasserstoff oder Fluor stehen,
   - R³, R⁴ und R⁶: gleich oder verschieden sind und unabhängig voneinander für Wasserstoff, Fluor, Chlor, Brom, Iod, Methyl, Ethyl, n- oder i-Propyl, n-, i-, s- oder t-Butyl, Trifluormethyl, Trichlormethyl oder Trifluorethyl stehen,
   - R⁵: für Wasserstoff, Fluor, Chlor, Brom, Cyano, Nitro, Methyl, Ethyl, n- oder i-Propyl, n-, i-, s- oder t-Butyl, Cyclopropyl, Methoxy, Ethoxy, Methylthio, Ethylthio, n- oder i-Propylthio, Trifluormethyl, Trichlormethyl, Trifluorethyl, Difluormethoxy, Trifluormethoxy, Difluorchlormethoxy, Trifluorethoxy, Di-fluormethylthio, Difluorchlormethylthio oder Trifluormethylthio steht,
   - R⁵: außerdem für Iod steht,
   wobei R³, R⁴, R⁵ und R⁶ nicht gleichzeitig für Wasserstoff stehen.
   Besonders bevorzugt sind Methylthiophencarboxanilide der Formel (I) sind, in welcher
   - R¹ und R²: gleich oder verschieden sind und unabhängig voneinander für Wasserstoff oder Fluor stehen,
   - R³, R⁴ und R⁶: gleich oder verschieden sind und unabhängig voneinander für Wasserstoff, Fluor, Chlor, Brom, Iod, Methyl oder Trifluormethyl stehen,
   - R⁵: für Wasserstoff, Fluor, Chlor, Brom, Methyl, Cyclopropyl, Methoxy, Methylthio, Trifluormethyl, Trichlormethyl, Trifluorethyl, Difluormethoxy, Trifluormethoxy, Difluorchlormethoxy, Trifluorethoxy, Difluormethylthio, Difluorchlormethylthio oder Trifluormethylthio steht,
   - R⁵: außerdem für Iod oder Cyano steht,
   wobei R³, R⁴, R⁵ und R⁶ nicht gleichzeitig für Wasserstoff stehen.
   Ganz besonders bevorzugt sind Verbindungen der Formel (I), in welcher R¹ und R² jeweils für Wasserstoff stehen.
   Ganz besonders bevorzugt sind Verbindungen der Formel (I), in welcher R¹ für Fluor und R² für Wasserstoff steht.
   Ganz besonders bevorzugt sind Verbindungen der Formel (I), in welcher R¹ für Wasserstoff und R² für Fluor steht.
   Ganz besonders bevorzugt sind Verbindungen der Formel (I), in welcher R³, R⁴ und R⁶ jeweils für Wasserstoff stehen und R⁵ nicht für Wasserstoff steht.
   Ganz besonders bevorzugt sind Verbindungen der Formel (I), in welcher R³ und R⁶ jeweils für Wasserstoff stehen und R⁴ und R⁵ jeweils nicht für Wasserstoff stehen.
   Ganz besonders bevorzugt sind Verbindungen der Formel (I), in welcher R⁴ und R⁶ jeweils für Wasserstoff stehen und R³ und R⁵ jeweils nicht für Wasserstoff stehen.
   Ganz besonders bevorzugt sind Verbindungen der Formel (I), in welcher R³ und R⁵ jeweils für Wasserstoff stehen und R⁴ und R⁶ jeweils nicht für Wasserstoff stehen.
   Weiterhin ganz besonders bevorzugt sind Verbindungen der Formel (I-a) 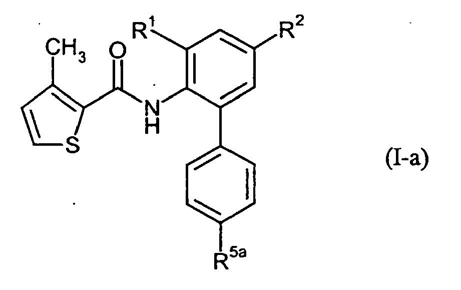 in welcher
   - R¹ und: R² die oben angegebenen Bedeutungen haben und
   - R^{5a}: für Fluor, Chlor, Brom, Methyl, Methylthio, Trifluormethyl, Trifluormethoxy, oder Trifluormethylthio steht,
   - R^{5a}: außerdem für Iod oder Cyano steht.

   Insbesondere ganz besonders bevorzugt sind Verbindungen der Formel (I-a), in welcher R¹ und R² jeweils für Wasserstoff stehen.
   Weiterhin ganz besonders bevorzugt sind Verbindungen der Formel (I-b) 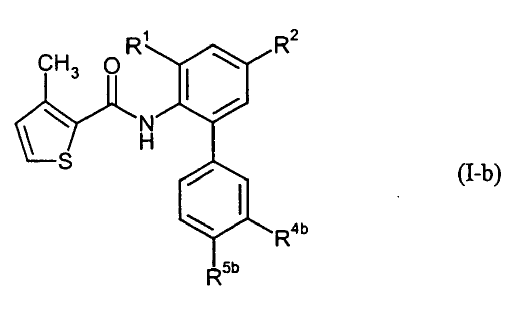 in welcher
   - R¹ und: R² die oben angegebenen Bedeutungen haben,
   - R^{4b}: für Fluor, Chlor, Brom, Methyl oder Trifluormethyl steht und
   - R^{5b}: für Fluor, Chlor, Brom, Methyl, Trifluormethyl, Trifluormethoxy oder Trifluormethylthio steht.

   Insbesondere ganz besonders bevorzugt sind Verbindungen der Formel (I-b), in welcher R¹ und R² jeweils für Wasserstoff stehen.
   Weiterhin ganz besonders bevorzugt sind Verbindungen der Formel (I-c) 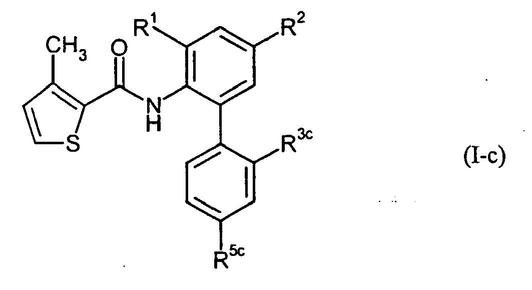 in welcher
   - R¹ und: R² die oben angegebenen Bedeutungen haben,
   - R^{3c}: für Fluor, Chlor, Brom oder Methyl steht und
   - R^{5c}: für Fluor, Chlor, Methyl, Trifluormethyl, Trifluormethoxy oder Trifluormethylthio steht.

   Insbesondere ganz besonders bevorzugt sind Verbindungen der Formel (I-c), in welcher R¹ und R² jeweils für Wasserstoff stehen.
   Weiterhin ganz besonders bevorzugt sind Verbindungen der Formel (I-d) 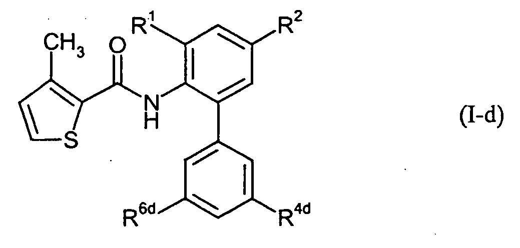 in welcher
   - R¹ und R²: die oben angegebenen Bedeutungen haben,
   - R^{4d} für: Fluor oder Chlor steht und
   - R^{6d} für: Fluor, Chlor, Trifluormethyl oder Trifluormethoxy steht.

   Insbesondere ganz besonders bevorzugt sind Verbindungen der Formel (I-d), in welcher R¹ und R² jeweils für Wasserstoff stehen.
   Die oben aufgeführten allgemeinen oder in Vorzugsbereichen aufgeführten Restedefinitionen bzw. Erläuterungen können auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können auch einzelne Definitionen entfallen.
   Gesättigte Kohlenwasserstoffreste wie Alkyl können, auch in Verbindung mit Heteroatomen, wie z.B. in Alkoxy, soweit möglich, jeweils geradkettig oder verzweigt sein.
   Durch Halogen substituierte Reste, z.B. Halogenalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.
   Verwendet man beispielsweise 3-Methyl-2-thiophencarbonylchlorid und 3'-Chlor-4'-fluor-1,1'-biphenyl-2-amin als Ausgangsstoffe sowie eine Base, so kann der Verlauf des erfindungsgemäßen Verfahrens (a) durch das folgende Formelschema veranschaulicht werden: 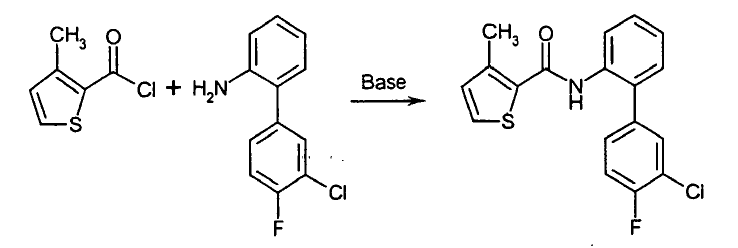
   Die zur Durchführung des erfindungsgemäßen Verfahrens (a) als Ausgangsstoffe benötigten Methylthiophencarbonsäurehalogenide sind durch die Formel (II) allgemein definiert. In dieser Formel (II) steht X¹ bevorzugt für Chlor.
   Die Methylthiophencarbonsäurehalogenide der Formel (II) sind bekannte erhältliche Laborchemikalien.
   Die weiterhin zur Durchführung des erfindungsgemäßen Verfahrens (a) als Ausgangsstoffe benötigten Anilinderivate sind durch die Formel (III) allgemein definiert. In dieser Formel (III) haben R¹, R², R³, R⁴, R⁵ und R⁶ bevorzugt bzw. besonders bevorzugt für diejenigen Bedeutungen, die bereits im Zusammenhang mit der Beschreibung der erfindungsgemäßen Verbindungen der Formel (I) für diese Reste als bevorzugt bzw. besonders bevorzugt angegeben wurden.
   Die Anilinderivate der Formel (III) sind bekannt und/oder lassen sich nach bekannten Methoden herstellen (vgl. z.B. Bull. Korean Chem. Soc. 2000, 21, 165-166; Chem. Pharm. Bull. 1992, 40, 240-4; JP 9-132567).
   Verwendet man beispielsweise *N*-(2-Iodphenyl)-3-methyl-2-thiophencarboxamid und 3-Chlor-4-fluorphenylboronsäure als Ausgangsstoffe sowie einen Katalysator und eine Base, so kann der Verlauf des erfindungsgemäßen Verfahrens (b) durch das folgende Formelschema veranschaulicht werden: 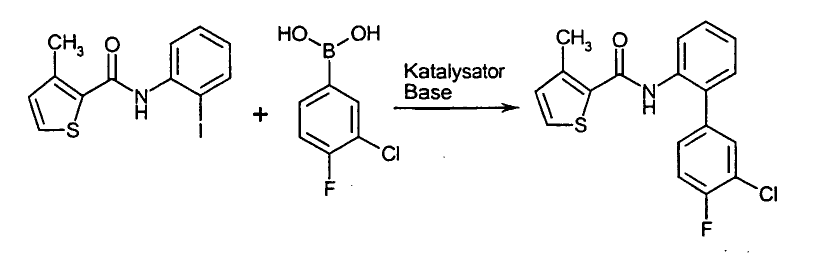
   Die zur Durchführung des erfindungsgemäßen Verfahrens (b) als Ausgangsstoffe benötigten Methylthiophencarboxhalogenanilide sind durch die Formel (IV) allgemein definiert. In dieser Formel (IV) stehen R¹ und R² bevorzugt bzw. besonders bevorzugt für diejenigen Bedeutungen, die bereits im Zusammenhang mit der Beschreibung der erfindungsgemäßen Verbindungen der Formel (I) für diese Reste als bevorzugt bzw. besonders bevorzugt angegeben wurden. X² steht bevorzugt für Brom oder Iod.
   Die Methylthiophencarboxhalogenanilide der Formel (IV) sind noch nicht bekannt. Sie sind neue chemische Verbindungen und ebenfalls Gegenstand der vorliegenden Anmeldung. Sie werden erhalten, indem man
c) Methylthiophencarbonsäurehalogenide der Formel (II) 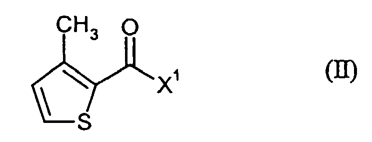 in welcher
   - X¹: für Halogen steht,
   mit 2-Bromanilin oder 2-Iodanilin gegebenenfalls in Gegenwart eines Säurebindemittels und gegebenenfalls in Gegenwart eines Verdünnungsmittels umsetzt.

Die zur Durchführung des erfindungsgemäßen Verfahrens (c) als Ausgangsstoffe benötigten Methylthiophencarbonsäurehalogenide der Formel (II) sind bereits oben im Zusammenhang mit dem erfindungsgemäßen Verfahren (a) beschrieben worden.

Die weiterhin zur Durchführung des erfindungsgemäßen Verfahrens (c) als Ausgangsstoffe benötigten Stoffe 2-Bromanilin und 2-Iodanilin sind bekannte Synthesechemikalien.

Die weiterhin zur Durchführung des erfindungsgemäßen Verfahrens (b) als Ausgangsstoffe benötigten Boronsäuren sind durch die Formel (V) allgemein definiert. In dieser Formel (V) stehen R³, R⁴, R⁵ und R⁶ bevorzugt bzw. besonders bevorzugt für diejenigen Bedeutungen, die bereits im Zusammenhang mit der Beschreibung der erfindungsgemäßen Verbindungen der Formel (I) für diese Reste als bevorzugt bzw. besonders bevorzugt angegeben wurden.

Boronsäuren der Formel (V) sind bekannte Synthesechemikalien. Sie können auch unmittelbar vor der Reaktion direkt aus Halogenbenzolderivaten und Boronsäureestem hergestellt und ohne Aufarbeitung weiter umgesetzt werden (siehe auch die Herstellungsbeispiele).

Als Verdünnungsmittel zur Durchführung der erfindungsgemäßen Verfahrens (a) und (c) kommen alle inerten organischen Lösungsmittel in Betracht. Hierzu gehören vorzugsweise aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Die erfindungsgemäßen Verfahren (a) und (c) werden gegebenenfalls in Gegenwart eines geeigneten Säurebindemittels durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a) und (c) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens (a) zur Herstellung der Verbindungen der Formel (I) setzt man pro Mol des Methylthiophencarbonsäurehalogenides der Formel (II) im allgemeinen 0,2 bis 5 Mol, vorzugsweise 0,5 bis 2 Mol an Anilinderivat der Formel (III) ein.

Zur Durchführung des erfindungsgemäßen Verfahrens (c) zur Herstellung der Verbindungen der Formel (IV) setzt man pro Mol des Methylthiophencarbonsäurehalogenides der Formel (II) im allgemeinen 0,2 bis 5 Mol, vorzugsweise 0,5 bis 2 Mol an 2-Bromanilin oder 2-Iodanilin ein.

Als Verdünnungsmittel zur Durchführung des erfindungsgemäßen Verfahrens (b) kommen alle inerten organischen Lösungsmittel in Betracht. Hierzu gehören vorzugsweise aliphatische, alicyclische oder aromatische Kohlenwasserstoffe, wie Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, s- oder t-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens (b) in einem größeren Bereich variiert werden. Im allgemeinen arbeitet man bei Temperaturen von 0°C bis 150°C, vorzugsweise bei Temperaturen von 20°C bis 110°C.

Das erfindungsgemäße Verfahren (b) wird gegebenenfalls in Gegenwart eines geeigneten Säurebindemittels durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, fluoride, phosphate, -carbonate oder -hydrogencarbonate, wie Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumphosphat, Kaliumphosphat, Kaliumfluorid, Cäsiumfluorid, Natriumcarbonat, Kaliumcarbonat, Kaliumhydrogencarbonat, Natriumhydrogencarbonat oder Cäsiumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (b) wird in Gegenwart eines Katalysators, wie beispielsweise eines Palladiumsalzes oder -komplexes, durchgeführt. Hierzu kommen vorzugsweise Palladiumchlorid, Palladiumacetat, Tetrakis-(triphenylphosphin)-palladium, Bis-(triphenylphosphin)-palladiumdichlorid oder 1,1'-Bis(diphenylphosphino)ferrocenpalladium(II)chlorid infrage.

Es kann auch ein Palladiumkomplex in der Reaktionsmischung erzeugt werden, wenn man ein Palladiumsalz und einen Komplexliganden, wie beispielsweise Triethylphosphan, Tri-tert-butylphosphan, Tricyclohexylphosphan, 2-(Dicyclohexylphosphan)biphenyl, 2-(Di-tert-butylphosphan)-biphenyl, 2-(Dicyclohexylphosphan)-2'-(N,N-dimethylamino)biphenyl, Triphenylphosphan, Tris-(o-tolyl)-phosphan, Natrium-3-(diphenylphosphino)benzolsulfonat, Tris-2-(Methoxyphenyl)-phosphan, 2,2'-Bis-(diphenylphosphan)-1,1'-binaphthyl, 1,4-Bis-(diphenylphosphan)-butan, 1,2-Bis-(diphenylphosphan)-ethan, 1,4-Bis-(dicyclohexylphosphan)-butan, 1,2-Bis-(dicyclohexylphosphan)-ethan, 2-(Dicyclohexylphosphan)-2'-(N,N-dimethylamino)-biphenyl, Bis(diphenylphosphino)ferrocen oder Tris-(2,4-tert-butylphenyl)-phosphit getrennt zur Reaktion zugibt.

Zur Durchführung des erfindungsgemäßen Verfahrens (b) zur Herstellung der Verbindungen der Formel (I) setzt man pro Mol des Methylthiophencarboxhalogenanilids der Formel (IV) im allgemeinen 1 bis 15 Mol, vorzugsweise 2 bis 8 Mol an Boronsäure der Formel (V) ein.

Die erfindungsgemäßen Verfahren (a), (b) und (c) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Pyrenophora-Arten, von Krankheiten im Wein-, Obst- und Gemüseanbau, wie beispielsweise gegen Venturia-, Sphaerotheca- und Podosphaera-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Emtegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxychinolinsulfat;
Acibenzolar-S-methyl; Aldimorph; Amidoflumet; Ampropylfos; Ampropylfos-potassium; Andoprim; Anilazine; Azaconazole; Azoxystrobin;
Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamin;
Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram;
Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon;
Edifenphos; Epoxiconazole; Ethaboxam; Ethirimol; Etridiazole;
Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox;
Guazatine; Hexachlorobenzene; Hexaconazole; Hymexazol;
Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione;
Kasugamycin; Kresoxim-methyl;
Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin;
Natamycin; Nicobifen; Nitrothal-isopropyl; Noviflumuron; Nuarimol;
Ofurace; Orysastrobin; Oxadixyl; Oxolinic acid; Oxpoconazole; Oxycarboxin; Oxyfenthiin;
Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine;
Quinconazole; Quinoxyfen; Quintozene; Simeconazole; Spiroxamine; Sulfur;
Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole;
Uniconazole; Validamycin A; Vinclozolin; Zineb; Ziram; Zoxamide;
(2S)-N-[2-[4-[[3-(4-Chlorphenyl)-2-propinyl]oxy]-3-methoxyphenyl]ethyl]-3-methyl- 2-[(methylsulfonyl)amino]-butanamid;
1-(1-Naphthalenyl)-1 H-pyrrol-2,5-dion;
2,3,5,6-Tetrachlor-4-(methylsulfonyl)-pyridin;
2-Amino-4-methyl-N-phenyl-5-thiazolcarboxamid;
2-Chlor-N-(2,3-dihydro-1,1,3-trimethyl-1H-inden-4-yl)-3-pyridincarboxamide; 3,4,5-Trichlor-2,6-pyridindicarbonitril;
Actinovate; cis-1-(4-Chlorphenyl)-2-(1H-1,2,4-triazol-1-yl)-cycloheptanol;
Methyl 1-(2,3-dihydro-2,2-dimethyl-1H-inden-1-yl)-1H-imidazol-5-carboxylat;
Monokaliumcarbonat; N-(6-Methoxy-3-pyridinyl)-cyclopropancarboxamid;
N-Butyl-8-(1,1-dimethylethyl)-1-oxaspiro[4.5]decan-3-amin;
Natriumtetrathiocarbonat;
sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin IR-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentylisomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439,
Eflusilanate, Emamectin, Emamectin-benzoate, Empenthrin (1R-isomer), Endosulfan, Entomopthora spp., EPN, Esfenvalerate; Ethiofencarb, Ethiprole, Ethion, Ethoprophos, Etofenprox, Etoxazole, Etrimfos,
Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin,
Japonilure, Kadethrin, Kempolyederviren, Kinoprene,
Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602, OK-9701, OK-9802, Omethoate, Oxamyl, Oxydemeton-methyl,
Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos, Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin; Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb,
Vamidothion, Vaniliprole, Verbutin, Verticillium lecanii,
WL-108477, WL-40027, YI-5201, YI-5301, YI-5302, XMC, Xylylcarb,
ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden.

Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise emiedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Emteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Emteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

### Herstellungsbeispiele

### Beispiel 1

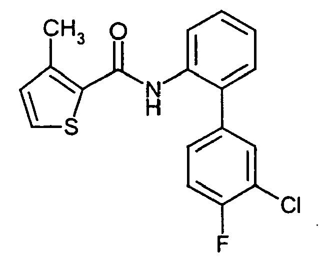

### Verfahren (a)

Zu einer Lösung von 0,288 g (1,3 mmol) 3'-Chlor-4'-fluor-1,1'-biphenyl-2-amin in 3 ml Tetrahydrofuran gibt man 0,36 ml (2,6 mmol) Triethylamin und eine Lösung von 0,25 g (1,56 mmol) 3-Methylthiophen-2-carbonylchlorid in 3 ml Tetrahydrofuran. Die Reaktionslösung wird 16 Stunden bei 60°C gerührt. Zur Aufarbeitung wird aufkonzentriert und mit Cyclohexan/Essigsäureethylester an Kieselgel chromatographiert.

Man erhält 0,45 g (99 % der Theorie) N-(3'-Chlor-4'-fluor-1,1'-biphenyl-2-yl)-3-methyl-2-thiophencarboxamid mit einem logP (pH 2,3) = 3,85.

### Beispiel 2

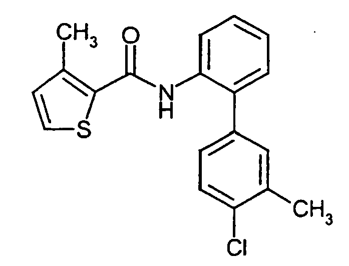

### Verfahren (b)

103 mg (0,5 mmol) 2-Chlor-5-bromtoluol, 162 mg (1,65 mmol) Kaliumacetat und 152 mg (0,6 mmol) Pinacoldiboronester werden in 8 ml entgastem Dimethylsulfoxid unter Sauerstoffausschluss gelöst und mit einer katalytischen Menge (37 mg) 1,1'-Bis(diphenylphosphino)ferrocenpalladium(II)chlorid versetzt. Das Reaktionsgemisch wird 2 Stunden bei 80-90°C gerührt. Nach dem Abkühlen gibt man 1,25 ml 2 molare Natriumcarbonatlösung, eine Lösung von 148 mg (0,5 mmol) N-(2-Bromphenyl)-3-methyl-2-thiophencarboxamid in 4 ml Dimethylsulfoxid, und weitere 37 mg 1,1'-Bis(diphenylphosphino)ferrocenpalladium(II)chlorid zur Reaktionsmischung hinzu und lässt weitere 16 Stunden bei 80-90°C rühren. Zur Aufarbeitung gibt man 2 ml Wasser und 8 ml Essigsäureethylester zu und trennt die organische Phase ab. Diese wird eingeengt und über Aktivkohle und Kieselsäure filtriert. Das Filtrat wird mit Cyclohexan/Essigsäureethylester (1:1) an Kieselgel chromatographiert.

Man erhält 79 mg (46 % der Theorie) N-(3'-Chlor-4'-methyl-1,1'-biphenyl-2-yl)-3-methyl-2-thiophencarboxamid mit dem logP (pH 2,3) = 4,41.

Analog den Beispielen 1 und 2, sowie entsprechend den Angaben in den allgemeinen Beschreibungen der Verfahren a) und b), werden die in der nachstehenden Tabelle 1 genannten Verbindungen der Formel (I) erhalten.

### Tabelle 1

| | | | | | | | |
|---|---|---|---|---|---|---|---|
| 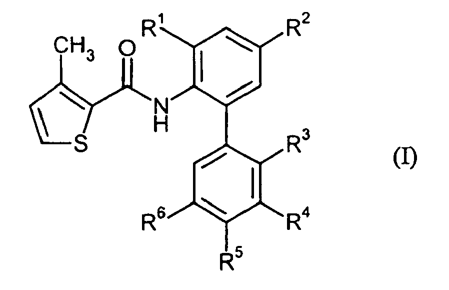 | | | | | | | |

| Bsp. | R¹ | R² | R³ | R⁴ | R⁵ | R⁶ | logP |
|---|---|---|---|---|---|---|---|
| 3 | H | H | H | H | Br | H | 4,12 |
| 4 | H | H | H | H | CF₃ | H | 4,06 |
| 5 | H | H | H | Cl | H | H | 3,97 |
| 6 | H | H | H | H | OCF₃ | H | 4,33 |
| 7 | H | H | H | H | SCH₃ | H | 4,03 |
| 8 | H | H | H | H | F | H | 3,65 |
| 9 | H | H | H | F | F | H | 3,55 |
| 10 | H | H | H | F | Cl | H | 3,85 |
| 11 | H | H | H | F | OCF₃ | H | 4,13 |
| 12 | H | H | H | CF₃ | OCF₃ | H | 4,37 |
| 13 | H | H | H | Cl | Cl | H | 4,18 |
| 14 | H | H | H | CF₃ | F | H | 3,85 |
| 15 | H | H | H | Cl | H | Cl | 4,32 |
| 16 | H | H | F | H | F | H | 3,41 |
| 17 | H | H | H | CF₃ | Cl | H | 4,13 |
| 18 | H | H | H | CF₃ | CH₃ | H | 4,27 |
| 19 | H | H | Cl | H | Cl | H | 5,76 |
| 20 | H | H | CH₃ | H | Cl | H | 4,51 |
| 21 | H | H | F | H | Cl | H | 3,75 |
| 22 | F | H | H | Cl | Cl | H | 3,71 |
| 23 | H | F | H | Cl | Cl | H | 4,10 |
| 24 | H | H | H | H | Cl | H | 3,87 |
| 25 | H | H | H | H | CN | H | 2,85 |
| 26 | H | H | H | F | CF₃ | H | 3,85 |
| 27 | H | H | H | CH₃ | F | H | 3,99 |
| 28 | H | H | H | F | H | F | 3,50 |
| 29 | H | H | H | H | I | H | 4,16 |
| 30 | H | H | H | F | CH₃ | H | 3,94 |
| 31 | H | H | H | Cl | CH₃ | H | 4,29 |
| 32 | H | H | F | H | CH₃ | H | 3,79 |
| 33 | H | H | H | Cl | CF₃ | H | 4,09 |

### Herstellung von Ausgangsstoffen der Formel (II)

### Beispiel (II-1)

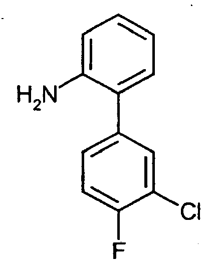

38,8 g (223 mmol) 3-Chlor-4-fluorphenylboronsäure, 40,6 g (186 mmol) 2-Iodanilin werden in 220 ml Toluol, 22 ml Ethanol und 45 ml einer 4 M Natriumhydrogencarbonatlösung unter Argon gelöst. Hierzu gibt man 4,3 g (4 mmol) Tetrakis(triphenylphosphin)palladium(0) und lässt die Reaktionslösung 16 Stunden bei 80°C unter Argon rühren. Die organische Phase wird abgetrennt, über Magnesiumsulfat getrocknet und aufkonzentriert. Der Rückstand wird mit Cyclohexan/Essigsäureethylester an Kieselgel chromatographiert.

Man erhält 22,5 g (48 % der Theorie) 3'-Chlor-4'-fluor-1,1'-biphenyl-2-amin mit dem logP (pH 2,3) = 3,01.

### Herstellung von Ausgangsstoffen der Formel (III)

### Beispiel (III-1)

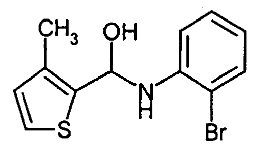

### Verfahren (c)

Zu einer Lösung von 5,36 g (0,031 mol) 2-Bromanilin in 80 ml Acetonitril gibt man 5,6 g (0,040 mol) Kaliumcarbonat und 6,0 g (0,037 mol) 3-Methylthiophen-2-carbonylchlorid. Die Reaktionslösung wird 16 Stunden unter Rückfluss erhitzt, anschließend filtriert und bei vermindertem Druck eingeengt. Der Rückstand wird mit Cyclohexan/Essigsäureethylester (2:1) an Kieselgel chromatographiert.

Man erhält 7,4 g (80 % der Theorie) N-(2-Bromphenyl)-3-methyl-2-thiophencarboxamid mit dem logP (pH 2,3) = 3,30.

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Podosphaera-Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle A Podosphaera-Test (Apfel) / protektiv**

| Wirkstoff | | Aufwandmenge an Wirkstoff in g/ha | Wirkungsgrad in % |
|---|---|---|---|
| 5 | 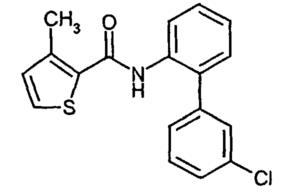 | 100 | 100 |
| 6 | 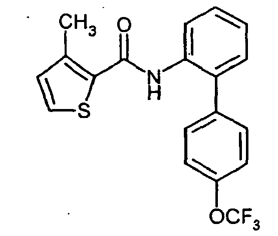 | 100 | 100 |
| 9 | 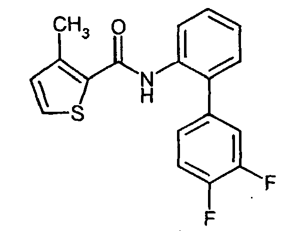 | 100 | 100 |
| 10 | 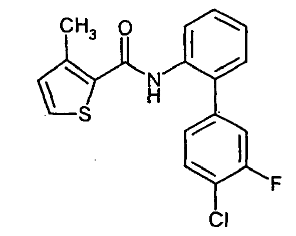 | 100 | 100 |
| 1 | 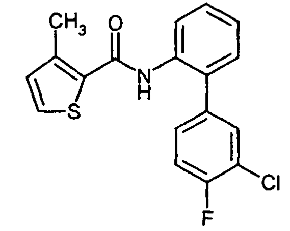 | 100 | 100 |
| 11 | 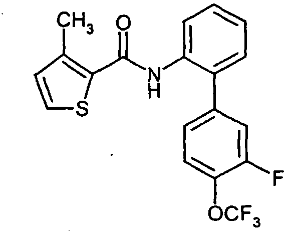 | 100 | 100 |
| 12 | 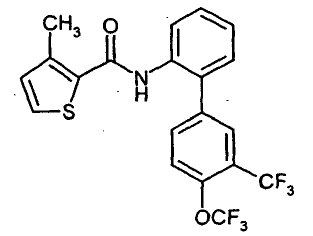 | 100 | 100 |
| 2 | 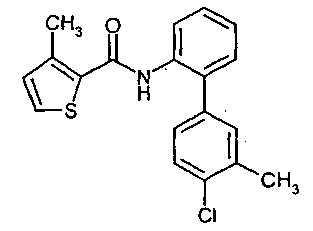 | 100 | 100 |
| 15 | 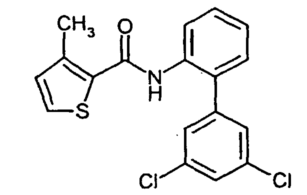 | 100 | 100 |
| 19 | 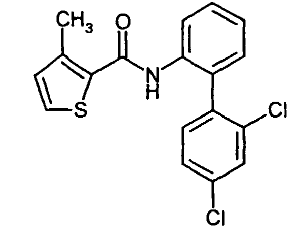 | 100 | 100 |
| 13 | 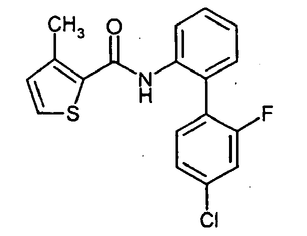 | 100 | 100 |

### Beispiel B

### Sphaerotheca-Test (Gurke) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Sphaerotheca fuliginea inokuliert. Die Pflanzen werden dann bei ca. 23°C und einer relativen Luftfeuchtigkeit von ca. 70 % im Gewächshaus aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle B Sphaerotheca-Test (Gurke) / protektiv**

| Wirkstoff | | Aufwandmenge an Wirkstoff in g/ha | Wirkungsgrad in % |
|---|---|---|---|
| 5 | 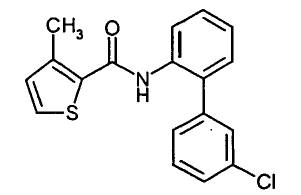 | 100 | 93 |
| 6 | 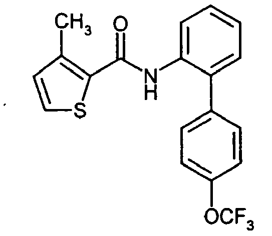 | 100 | 100 |
| 9 | 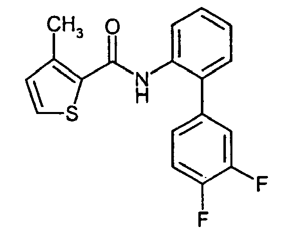 | 100 | 100 |
| 10 | 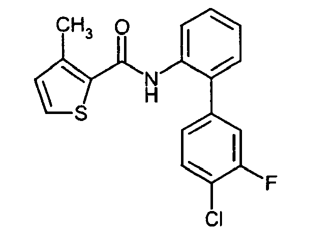 | 100 100 | 100 100 |
| 1 | 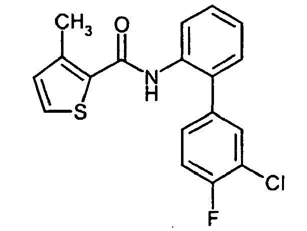 | 100 | 100 |
| 11 | 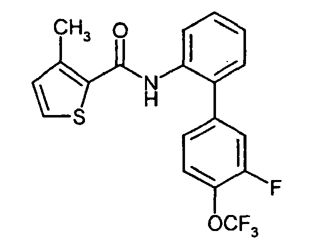 | 100 | 100 |
| 12 | 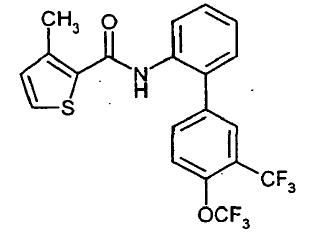 | 100 | 100 |
| 15 | 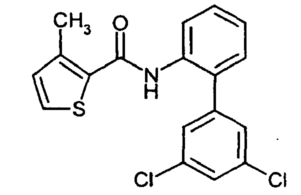 | 100 | 100 |
| 19 | 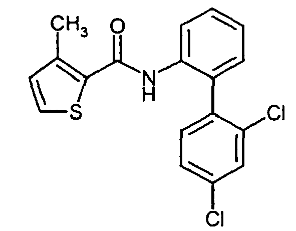 | 100 | 96 |
| 13 | 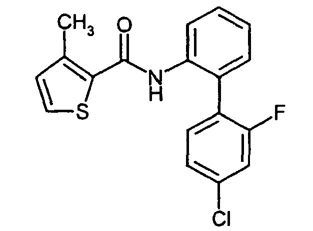 | 100 | 100 |

### Beispiel C

### Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle C Venturia - Test (Apfel) / protektiv**

| Wirkstoff | | Aufwandmenge an Wirkstoff in g/ha | Wirkungsgrad in % |
|---|---|---|---|
| 5 | 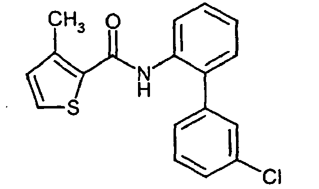 | 100 | 100 |
| 9 | 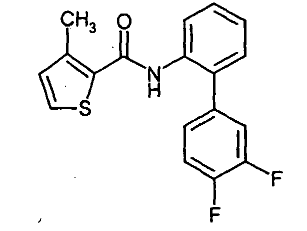 | 100 | 100 |
| 10 | 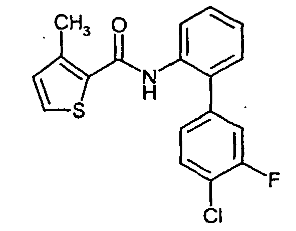 | 100 | 100 |
| 1 | 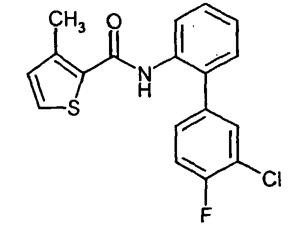 | 100 100 | 100 100 |
| 11 | 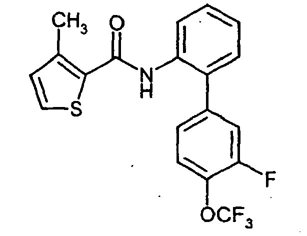 | 100 | 100 |
| 2 | 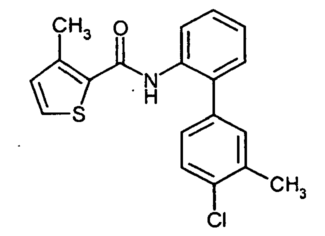 | 100 | 100 |
| 15 | 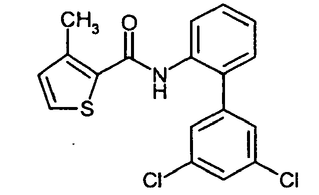 | 100 | 97 |
| 19 | 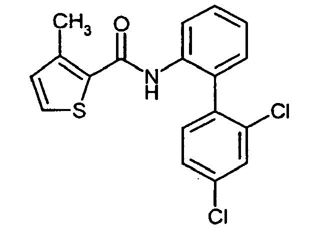 | 100 | 99 |
| 13 | 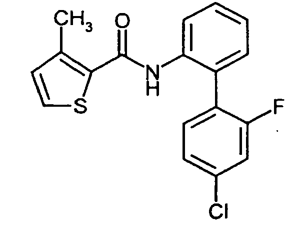 | 100 | 100 |

### Beispiel D

### Alternaria-Test (Tomate) / protektiv

| | |
|---|---|
| Lösungsmittel: | 49 Gewichtsteile N,N-Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von Alternaria solani inokuliert und stehen dann 24 h bei 100 % relativer Feuchte und 20°C. Anschließend stehen die Pflanzen bei 96 % relativer Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle D Alternaria-Test (Tomate) / protektiv**

| Wirkstoff | | Aufwandmenge an Wirkstoff in g/ha | Wirkungsgrad in % |
|---|---|---|---|
| 3 | 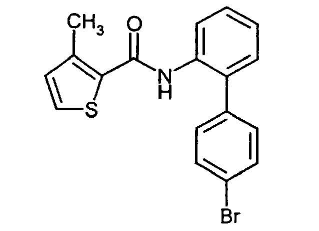 | 750 | 100 |
| 5 | 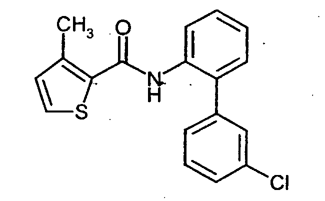 | 750 | 100 |
| 6 | 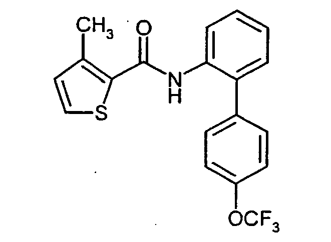 | 750 | 100 |
| 8 | 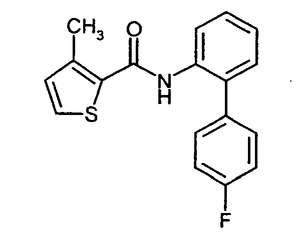 | 750 | 100 |
| 9 | 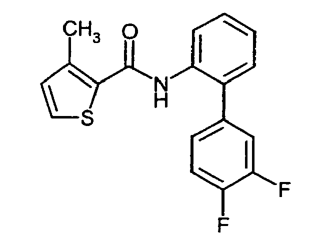 | 750 | 100 |
| 10 | 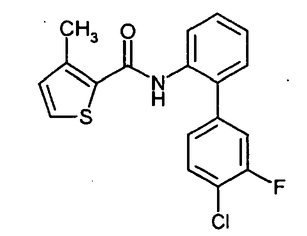 | 750 | 100 |

### Beispiel E

### Pyrenophora teres-Test (Gerste) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegeben Aufwandmenge besprüht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und relativen Luftfeuchtigkeit von ca. 80 % aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle E Pyrenophora teres-Test (Gerste) / protektiv**

| Wirkstoff | | Aufwandmenge an Wirkstoff in g/ha | Wirkungsgrad in % |
|---|---|---|---|
| 8 | 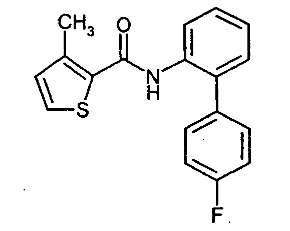 | 500 | 80 |
| 9 | 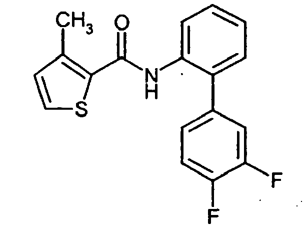 | 500 | 100 |
| 13 | 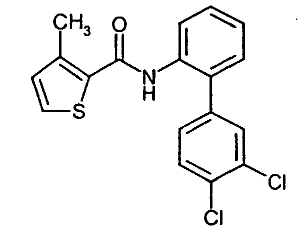 | 500 500 | 94 94 |

## Patentansprüche

1. Methylthiophenearboacanilide der Formel (I) 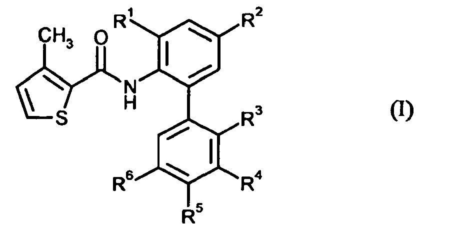 in welcher
R¹ und R² gleich oder verschieden sind und unabhängig voneinander für Wasserstoff oder Fluor stehen,
R³, R⁴ und R⁶ gleich oder verschieden sind und unabhängig voneinander für Wasserstoff, Halogen, C₁-C₆-Alkyl oder C₁-C₄-Halogenalkyl mit 1 bis 5 Halogenatomen stehen,
R⁵ für Wasserstoff, Halogen, Cyano, Nitro, C₁-C₆-Alkyl, C₂-C₆-Alkenyl, C₃-C₆-Cycloalkyl, C₁-C₄-Alkoxy, C₁-C₄-Alkylthio, C₁-C₄-Alkylsulfonyl, C₁-C₄-Halogenalkyl mit 1 bis 5 Halogenatomen, C₁-C₄-Halogenalkoxy mit 1 bis 5 Halogenatomen, C₁-C₄₋Halogenalkylthio mit 1 bis 5 Halogenatomen oder C₁-C₄-Halogenalkylsulfonyl mit 1 bis 5 Halogenatomen steht,
wobei R³, R⁴, R⁵ und R⁶ nicht gleichzeitig für Wasserstoff stehen.

2. Methylthiophencarboxanilide der Formel (I) gemäß Anspruch 1, in welcher
R¹ und R² gleich oder verschieden sind und unabhängig voneinander für Wasserstoff oder Fluor stehen,
R³, R⁴ und R⁶ gleich oder verschieden sind und unabhängig voneinander für Wasserstoff, Fluor, Chlor, Brom, Iod, Methyl, Ethyl, n- oder i-Propyl, n-, i-, s- oder t-Butyl, Trifluormethyl, Trichlormethyl oder Trifluorethyl stehen,
R⁵ für Wasserstoff, Fluor, Chlor, Brom, Cyano, Nitro, Methyl, Ethyl, n- oder i-Propyl, n-, i-, s- oder t-Butyl, Cyclopropyl, Methoxy, Ethoxy, Methylthio, Ethylthio, n- oder i-Propylthio, Trifluormethyl, Trichlormethyl, Trifluorethyl, Difluormethoxy, Trifluormethoxy, Difluorchlormethoxy, Trifluorethoxy, Difluormethylthio, Difluorchlormethylthio oder Trifluormethylthio steht,
R⁵ außerdem für Iod steht,
wobei R³, R⁴, R⁵ und R⁶ nicht gleichzeitig für Wasserstoff stehen.

3. Methylthiophencarboxanilide der Formel (I) gemäß Anspruch 1, in welcher
R¹ und R² gleich oder verschieden sind und unabhängig voneinander für Wasserstoff oder Fluor stehen,
R³, R⁴ und R⁶ gleich oder verschieden sind und unabhängig voneinander für Wasserstoff, Fluor, Chlor, Brom, Iod, Methyl oder Trifluormethyl stehen,
R⁵ für Wasserstoff, Fluor, Chlor, Brom, Methyl, Cyclopropyl, Methoxy, Methylthio, Trifluormethyl, Trichlormethyl, Trifluorethyl, Difluormethoxy, Trifluormethoxy, Difluorchlormethoxy, Trifluorethoxy, Difluormethylthio, Difluorchlormethylthio oder Trifluormethylthio steht,
R⁵ außerdem für Iod oder Cyano steht,
wobei R³, R⁴, R⁵ und R⁶ nicht gleichzeitig für Wasserstoff stehen.

4. Verfahren zum Herstellen von Methylthiophencarboxaniliden der Formel (I) gemäß Anspruch 1, **dadurch gekennzeichnet, dass** man
a) Methylthiophencarbonsäurehalogenide der Formel (II) 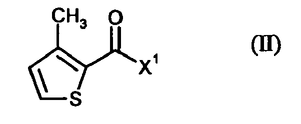 in welcher
X¹ für Halogen steht,
mit einem Anilinderivat der Formel (III) 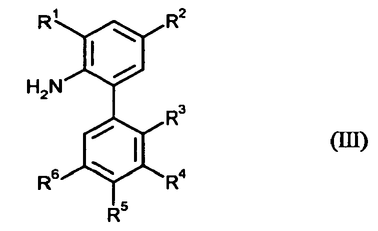 in welcher R¹, R², R³, R⁴, R⁵ und R⁶ die in Anspruch 1 angegebenen Bedeutungen haben,
gegebenenfalls in Gegenwart eines Säurebindemittels und gegebenenfalls in Gegenwart eines Verdünnungsmittels umsetzt, oder
b) Methylthiophencarboxhalogenanilide der Formel (IV) 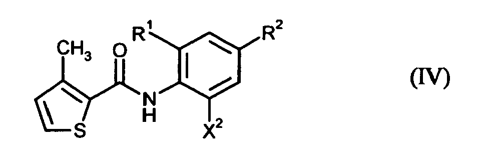 in welcher
R¹ und R² die in Anspruch 1 angegebenen Bedeutungen haben und
X² für Brom oder Iod steht,
mit einer Boronsäure der Formel (V) 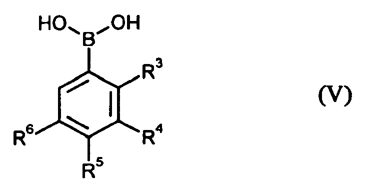 in welcher
R³, R⁴, R⁵ und R⁶ die in Anspruch 1 angegebenen Bedeutungen haben,
in Gegenwart eines Katalysators, gegebenenfalls in Gegenwart eines Säurebindemittels und gegebenenfalls in Gegenwart eines Verdünnungsmittels umsetzt.

5. Mittel zum Bekämpfen unerwünschter Mikroorganismen, **gekennzeichnet durch** einen Gehalt an mindestens einem Methylthiophencarboxanilid der Formel (I) gemäß Anspruch 1 neben Streckmitteln und/oder oberflächen-aktiven Stoffen.

6. Verwendung von Methylthiophencarboxaniliden der Formel (I) gemäß Anspruch 1 zum Bekämpfen unerwünschter Mikroorganismen.

7. Verfahren zum Bekämpfen unerwünschter Mikroorganismen, **dadurch gekennzeichnet, dass** man Methylthiophencarboxanilide der Formel (I) gemäß Anspruch 1 auf die Mikroorganismen und/oder deren Lebensraum ausbringt.

8. Verfahren zum Herstellen von Mitteln zum Bekämpfen unerwünschter Mikroorganismen, **dadurch gekennzeichnet, dass** man Methylthiophencarboxanilide der Formel (I) gemäß Anspruch 1 mit Streckmitteln und/oder oberflächenaktiven Stoffen vermischt.

9. Methylthiophencarboxhalogenanilide der Formel (IV) 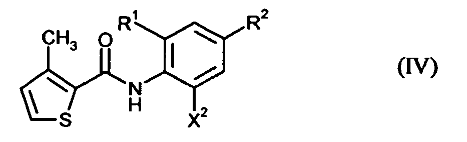 in welcher
R¹ und R² die in Anspruch 1 angegebenen Bedeutungen haben und
X² für Brom oder Iod steht.

10. Verbindungen der Formel (I-a) 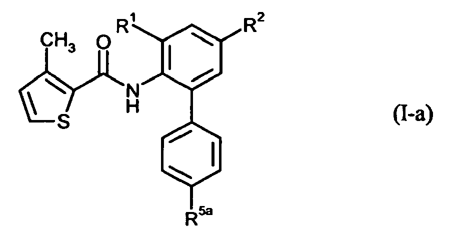 in welcher
R¹ und R² gleich oder verschieden sind und unabhängig voneinander für Wasserstoff oder Fluor stehen,
R^{5a} für Fluor, Chlor, Brom, Iod, Cyano, Methyl, Methylthio, Trifluormethyl, Trifluormethoxy, oder Trifluormethylthio steht.

11. Verbindungen der Formel (I-b) 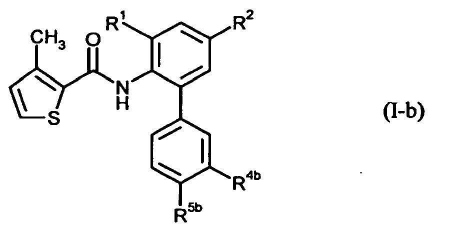 in welcher
R¹ und R² gleich oder verschieden sind und unabhängig voneinander für Wasserstoff oder Fluor stehen,
R^{4b} für Fluor, Chlor, Brom, Methyl oder Trifluormethyl steht und
R^{5b} für Fluor, Chlor, Brom, Methyl, Trifluormethyl, Trifluormethoxy oder Trifluormethylthio steht.

12. Verbindungen der Formel (I-c) 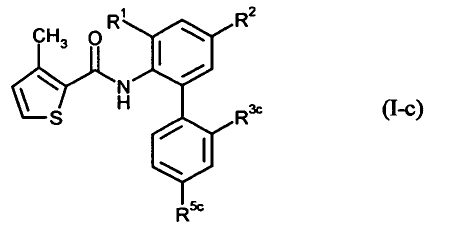 in welcher
R¹ und R² gleich oder verschieden sind und unabhängig voneinander für Wasserstoff oder Fluor stehen,
R^{3c} für Fluor, Chlor, Brom oder Methyl steht und
R^{5c} für Fluor, Chlor, Methyl, Trifluormethyl, Trifluormethoxy oder Trifluormethylthio steht.

13. Verbindungen der Formel (I-d) 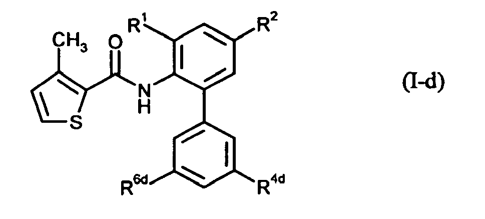 in welcher
R¹ und R² gleich oder verschieden sind und unabhängig voneinander für Wasserstoff oder Fluor stehen,
R^{4d} für Fluor oder Chlor steht und
R^{6d} für Fluor, Chlor, Trifluormethyl oder Trifluormethoxy steht.

14. Verbindungen der Formel (I-a) gemäß Anspruch 10, der Formel (I-b) gemäß Anspruch 11, der Formel (I-c) gemäß Anspruch 12 und der Formel (I-d) gemäß Anspruch 13, in welchen R¹ und R² jeweils für Wasserstoff stehen.

## Claims

1. Methylthiophenecarboxanilides of the formula (I) 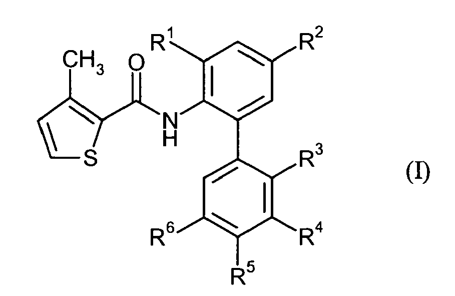 in which
R¹ and R² are identical or different and independently of one another represent hydrogen or fluorine,
R³, R⁴ and R⁶ are identical or different and independently of one another represent hydrogen, halogen, C₁-C₆-alkyl or C₁-C₄-haloalkyl having 1 to 5 halogen atoms,
R⁵ represents hydrogen, halogen, cyano, nitro, C₁-C₆-alkyl, C₂-C₆₋alkenyl, C₃-C₆-cycloalkyl, C₁-C₄-alkoxy, C₁-C₄-alkylthio, C₁-C₄₋alkylsulfonyl, C₁-C₄-haloalkyl having 1 to 5 halogen atoms, C₁-C₄₋haloalkoxy having 1 to 5 halogen atoms, C₁-C₄-haloalkylthio having 1 to 5 halogen atoms or C₁-C₄-haloalkylsulfonyl having 1 to 5 halogen atoms,
where R³, R⁴, R⁵ and R⁶ do not simultaneously represent hydrogen.

2. Methylthiophenecarboxanilides of the formula (I) according to Claim 1 in which
R¹ and R² are identical or different and independently of one another represent hydrogen or fluorine,
R³, R⁴ and R⁶ are identical or different and independently of one another represent hydrogen, fluorine, chlorine, bromine, iodine, methyl, ethyl, n- or i-propyl, n-, i-, s- or t-butyl, trifluoromethyl, trichloromethyl or trifluoroethyl,
R⁵ represents hydrogen, fluorine, chlorine, bromine, cyano, nitro, methyl, ethyl, n- or i-propyl, n-, i-, s- or t-butyl, cyclopropyl, methoxy, ethoxy, methylthio, ethylthio, n- or i-propylthio, trifluoromethyl, trichloromethyl, trifluoroethyl, difluoromethoxy, trifluoromethoxy, difluorochloromethoxy, trifluoroethoxy, difluoromethylthio, difluorochloromethylthio or trifluoromethylthio,
R⁵ furthermore represents iodine,
where R³, R⁴, R⁵ and R⁶ do not simultaneously represent hydrogen.

3. Methylthiophenecarboxanilides of the formula (I) according to Claim 1 in which
R¹ and R² are identical or different and independently of one another represent hydrogen or fluorine,
R³, R⁴ and R⁶ are identical or different and independently of one another represent hydrogen, fluorine, chlorine, bromine, iodine, methyl or trifluoromethyl,
R⁵ represents hydrogen, fluorine, chlorine, bromine, methyl, cyclopropyl, methoxy, methylthio, trifluoromethyl, trichloromethyl, trifluoroethyl, difluoromethoxy, trifluoromethoxy, difluorochloromethoxy, trifluoroethoxy, difluoromethylthio, difluorochloromethylthio or trifluoromethylthio,
R⁵ furthermore represents iodine or cyano,
where R³, R⁴, R⁵ and R⁶ do not simultaneously represent hydrogen.

4. Process for preparing methylthiophenecarboxanilides of the formula (I) according to Claim 1, **characterized in that**
a) methylthiophenecarbonyl halides of the formula (II) 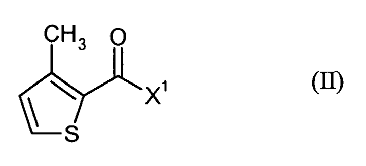 in which
X¹ represents halogen,
are reacted with an aniline derivative of the formula (III) 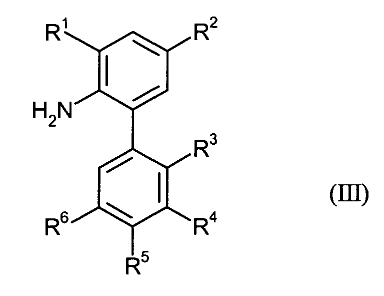 in which
R¹, R², R³, R⁴, R⁵ and R⁶ are as defmed in Claim 1,
if appropriate in the presence of an acid binder and if appropriate in the presence of a diluent, or
b) methylthiophenecarboxhalogenanilides of the formula (IV) 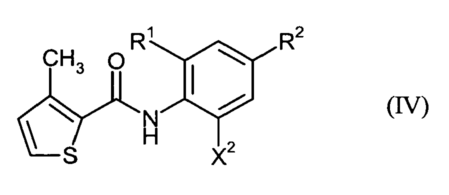 in which
R¹ and R² are as defined in Claim 1 and
X² represents bromine or iodine,
are reacted with a boronic acid of the formula (V) 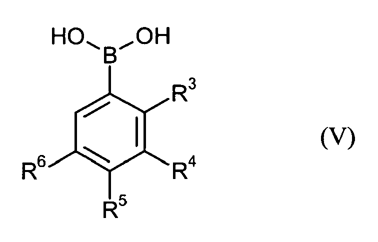 in which
R³, R⁴, R⁵ and R⁶ are as defined in Claim 1,
in the presence of a catalyst, if appropriate in the presence of an acid binder and if appropriate in the presence of a diluent.

5. Composition for controlling unwanted micro-organisms, **characterized in that** they comprise at least one methylthiophenecarboxanilide of the formula (I) according to Claim 1, in addition to extenders and/or surfactants.

6. Use of methylthiophenecarboxanilides of the formula (I) according to Claim 1 for controlling unwanted micro-organisms.

7. Method for controlling unwanted micro-organisms, **characterized in that** methylthiophenecarboxanilides of the formula (I) according to Claim 1 are applied to the micro-organisms and/or their habitat.

8. Method for preparing compositions for controlling unwanted micro-organisms, **characterized in that** methylthiophenecarboxanilides of the formula (I) according to Claim 1 are mixed with extenders and/or surfactants.

9. Methylthiophenecarboxhaloanilides of the formula (IV) 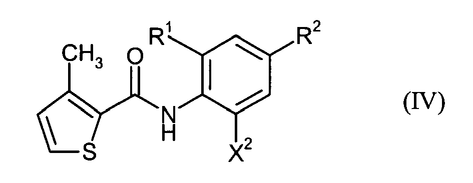 in which
R¹ and R² are as defined in Claim 1 and
X² represents bromine or iodine.

10. Compounds of the formula (I-a) 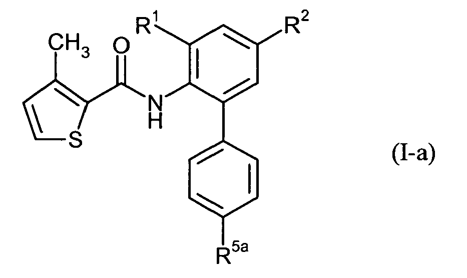 in which
R¹ and R² are identical or different and independently of one another represent hydrogen or fluorine,
R^{5a} represents fluorine, chlorine, bromine, iodine, cyano, methyl, methylthio, trifluoromethyl, trifluoromethoxy, or trifluoromethylthio.

11. Compounds of the formula (I-b) 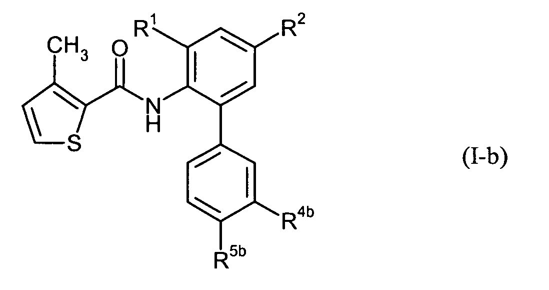 in which
R¹ and R² are identical or different and independently of one another represent hydrogen or fluorine,
R^{4b} represents fluorine, chlorine, bromine, methyl or trifluoromethyl and
R^{5b} represents fluorine, chlorine, bromine, methyl, trifluoromethyl, trifluoromethoxy or trifluoromethylthio.

12. Compounds of the formula (I-c) 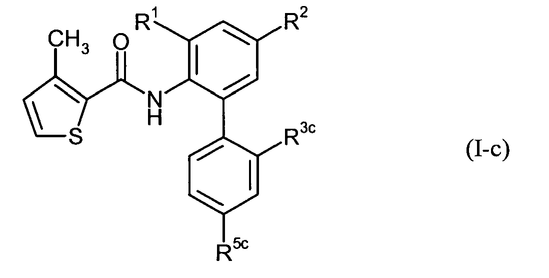 in which
R¹ and R² are identical or different and independently of one another represent hydrogen or fluorine,
R^{3c} represents fluorine, chlorine, bromine or methyl and
R^{5c} represents fluorine, chlorine, methyl, trifluoromethyl, trifluoromethoxy or trifluoromethylthio.

13. Compounds of the formula (I-d) 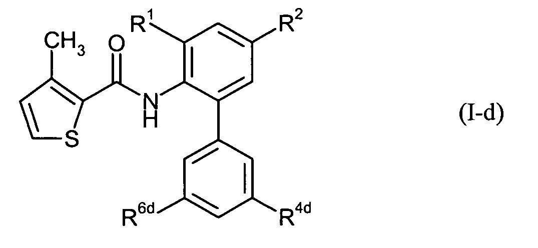 in which
R¹ and R² are identical or different and independently of one another represent hydrogen or fluorine,
R^{4d} represents fluorine or chlorine and
R^{6d} represents fluorine, chlorine, trifluoromethyl or trifluoromethoxy.

14. Compounds of the formula (I-a) according to Claim 10, of the formula (I-b) according to Claim 11, of the formula (I-c) according to Claim 12 and of the formula (I-d) according to Claim 13, in which R₁ and R₂ each represent hydrogen.

## Revendications

1. Méthylthiophènecarboxanilides de formule (I) 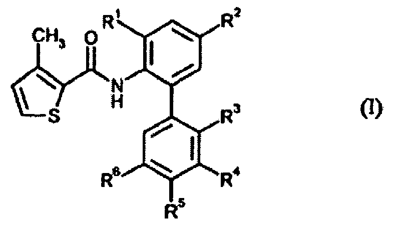 dans laquelle
R¹ et R² sont identiques ou différents et représentent, indépendamment l'un de l'autre, l'hydrogène ou le fluor,
R³ R⁴ et R⁶ sont identiques ou différents et représentent, indépendamment les uns des autres, l'hydrogène, un halogène, un reste alkyle en C₁ à C₆ ou un reste halogénalkyle en C₁ à C₄ ayant 1 à 5 atomes d'halogène,
R⁵ représente l'hydrogène, un halogène, un groupe cyano, nitro, un reste alkyle en C₁ à C₆, alcényle en C₂ à C₆, cycloalkyle en C₃ à C₆, alkoxy en C₁ à C₄, alkylthio en C₁ à C₄, alkylsulfonyle en C₁ à C₄, halogénalkyle en C₁ à C₄ ayant 1 à 5 atomes d'halogène, halogénalkoxy en C₁ à C₄ ayant 1 à 5 atomes d'halogène, halogénalkylthio en C₁ à C₄ ayant 1 à 5 atomes d'halogène ou halogénalkylsulfonyle en C₁ à C₄ ayant 1 à 5 atomes d'halogène,
R³, R⁴, R⁵ et R⁶ ne représentant pas en même temps l'hydrogène.

2. Méthylthiophènecarboxanilides de formule (I) suivant la revendication 1, formule dans laquelle
R¹ et R² sont identiques ou différents et représentent, indépendamment l'un de l'autre, l'hydrogène ou le fluor,
R³, R⁴ et R⁶ sont identiques ou différents et représentent, indépendamment les uns des autres, l'hydrogène, le fluor, le chlore, le brome, l'iode, un reste méthyle, éthyle, n-propyle, isopropyle, n-butyle, isobutyle, sec.-butyle, tertiobutyle, trifluorométhyle, trichlorométhyle ou trifluoréthyle,
R⁵ représente l'hydrogène, le fluor, le chlore, le brome, un groupe cyano, nitro, un reste méthyle, éthyle, n-propyle, isopropyle, n-butyle, isobutyle, sec.-butyle, tertiobutyle, cyclopropyle, méthoxy, éthoxy, méthylthio, éthylthio, n-propylthio, isopropylthio, trifluorométhyle, trichlorométhyle, trifluoréthyle, difluorométhoxy, trifluorométhoxy, difluorochlorométhoxy, trifluoréthoxy, difluorométhylthio, difluorochlorométhylthio ou trifluorométhylthio,
R⁵ représente en outre l'iode,
R³, R⁴, R⁵ et R⁶ ne représentant pas en même temps l'hydrogène.

3. Méthylthiophènecarboxanilides de formule (I) suivant la revendication 1, formule dans laquelle
R¹ et R² sont identiques ou différents et représentent, indépendamment l'un de l'autre, l'hydrogène ou le fluor,
R³, R⁴ et R⁶ sont identiques ou différents et représentent, indépendamment les uns des autres, l'hydrogène, le fluor, le chlore, le brome, l'iode, un reste méthyle ou trifluorométhyle,
R⁵ représente l'hydrogène, le fluor, le chlore, le brome, un reste méthyle, cyclopropyle, méthoxy, méthylthio, trifluorométhyle, trichlorométhyle, trifluoréthyle, difluorométhoxy, trifluorométhoxy, difluorochlorométhoxy, trifluoréthoxy, difluorométhylthio, difluorochlorométhylthio ou trifluorométhylthio,
R⁵ représente en outre l'iode ou le groupe cyano,
R³ R⁴ R⁵ et R⁶ ne représentant pas en même temps l'hydrogène.

4. Procédé de production de méthylthiophènecarboxanilides de formule (I) suivant la revendication 1, **caractérisé en ce que** :
(a) on fait réagir des halogénures d'acide méthylthiophènecarboxylique de formule (II) 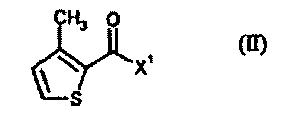 dans laquelle
X₁ représente un halogène,
avec un dérivé d'aniline de formule (III) 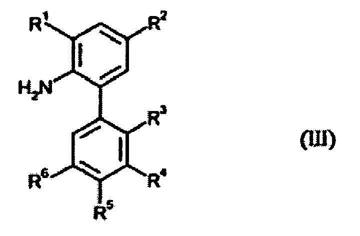 dans laquelle
R¹, R², R³, R⁴, R⁵ et R⁶ ont les définitions indiquées dans la revendication 1,
le cas échéant en présence d'un accepteur d'acide et en présence éventuelle d'un diluant, ou bien
(b) on fait réagir des méthylthiophènecarboxhalogénanilides de formule (IV) 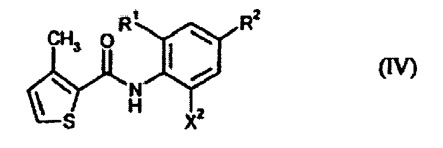 dans laquelle
R¹ et R² ont les définitions indiquées dans la revendication 1, et
X² représente le brome ou l'iode,
avec un acide boronique de formule (V) 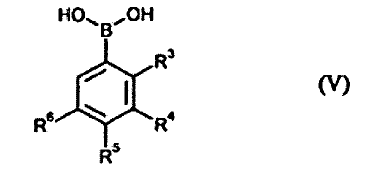 dans laquelle
R³, R⁴, R⁵ et R⁶ ont les définitions indiquées dans la revendication 1,
en présence d'un catalyseur, éventuellement en présence d'un accepteur d'acide et, le cas échéant, en présence d'un diluant.

5. Composition destinée à combattre des micro-organismes indésirables, **caractérisée par** une teneur en au moins un méthylthiophènecarboxanilide de formule (I) suivant la revendication 1, à côté de diluants et/ou d'agents tensioactifs.

6. Utilisation de méthylthiophènecarboxanilides de formule (I) suivant la revendication 1, pour combattre des micro-organismes indésirables.

7. Procédé pour combattre des micro-organismes indésirables, **caractérisé en qu'**on épand des méthylthiophènecarboxanilides de formule (I) suivant la revendication 1 sur les micro-organismes et/ou sur leur milieu.

8. Procédé de préparation de compositions destinées à combattre des micro-organismes indésirables, **caractérisé en ce qu'**on mélange des méthylthiophènecarboxanilides de formule (I) suivant la revendication 1 avec des diluants et/ou des agents tensioactifs.

9. Méthylthiophènecarboxhalogénanilides de formule (IV), 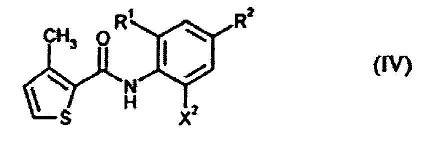 dans laquelle
R¹ et R² ont les définitions indiquées dans la revendication 1, et
X² représente le brome ou l'iode.

10. Composés de formule (I-a) 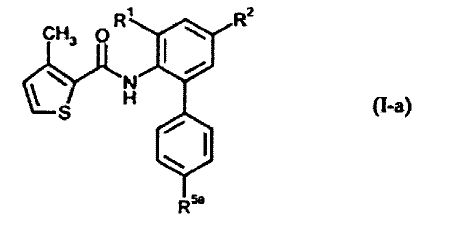 dans laquelle
R¹ et R² sont identiques ou différents et représentent, indépendamment l'un de l'autre, l'hydrogène ou le fluor,
R^{5a} représente le fluor, le chlore, le brome, l'iode, un groupe cyano, un reste méthyle, méthylthio, trifluorométhyle, trifluorométhoxy ou trifluorométhylthio.

11. Composés de formule (I-b) 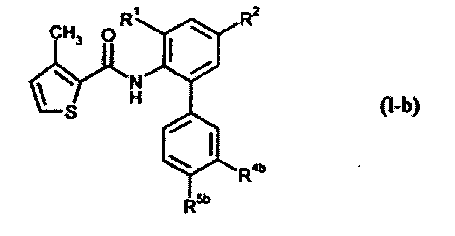 dans laquelle
R¹ et R² sont identiques ou différents et représentent, indépendamment l'un de l'autre, l'hydrogène ou le fluor,
R^{4b} représente le fluor, le chlore, le brome, un reste méthyle ou trifluorométhyle et
R^{5b} représente le fluor, le chlore, le brome, un reste méthyle, trifluorométhyle, trifluorométhoxy ou trifluorométhylthio.

12. Composés de formule (I-c) 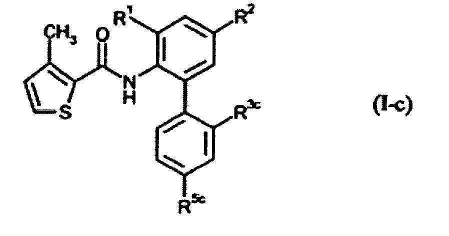 dans laquelle
R¹ et R² sont identiques ou différents et représentent, indépendamment l'un de l'autre, l'hydrogène ou le fluor,
R^{3c} représente le fluor, le chlore, le brome ou un reste méthyle, et
R^{5c} représente le fluor, le chlore, un reste méthyle, trifluorométhyle, trifluorométhoxy ou trifluorométhylthio.

13. Composés de formule (I-d) 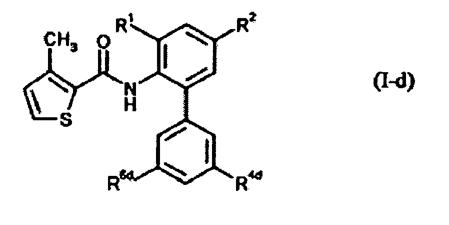 dans laquelle
R¹ et R² sont identiques ou différents et représentent, indépendamment l'un de l'autre, l'hydrogène ou le fluor,
R^{4d} représente le fluor ou le chlore, et
R^{6d} représente le fluor, le chlore, un reste trifluorométhyle ou trifluorométhoxy.

14. Composés de formule (I-a) suivant la revendication 10, de formule (I-b) suivant la revendication 11, de formule (I-c) suivant la revendication 12 et de formule (I-d) suivant la revendication 13, formules dans lesquelles R¹ et R² représentent chacun l'hydrogène.
